(19) Europäisches Patentamt
European Patent Office
Office européen des brevets

(11) Veröffentlichungsnummer: **0 469 432 A1**

(12) # EUROPÄISCHE PATENTANMELDUNG

(21) Anmeldenummer: **91112291.9**

(51) Int. Cl.⁵: **C07D 513/04**

(22) Anmeldetag: **23.07.91**

(30) Priorität: **03.08.90 DE 4024692**

(43) Veröffentlichungstag der Anmeldung: **05.02.92 Patentblatt 92/06**

(84) Benannte Vertragsstaaten: **CH DE FR GB IT LI**

(71) Anmelder: **MERCK PATENT GESELLSCHAFT MIT BESCHRÄNKTER HAFTUNG**
**Frankfurter Strasse 250**
**W-6100 Darmstadt(DE)**

(72) Erfinder: **Casutt, Michael, Dr.**
**Nelkenweg 19**
**W-6148 Heppenheim(DE)**
Erfinder: **Schwarz, Michael, Dr.**
**Hanfgraben 20**
**W-6080 Gross-Gerau 3(DE)**

(54) **Verfahren zur Herstellung von Cyanhydantionen.**

(57) Die Erfindung betrifft ein verbessertes Verfahren zur Herstellung von chiralen Cyanhydantoinen der Formel I,

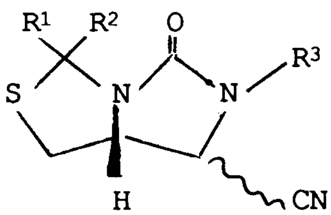

I

wobei

$R^1$ und $R^2$ jeweils unabhängig voneinander H, unsubstituiertes oder substituiertes Alkyl, Cycloalkyl, Aryl, Aralkyl oder Heteroaryl oder zusammengenommen unsubstituiertes Alkylen oder Heteroalkylen, und

$R^3$ H, unsubstituiertes oder durch eine oder mehrere Alkyl-, Alkoxy oder Alk-2-enyl-Gruppen substituiertes Benzyl oder $SiR^4R^5R^6$,

worin

$R^4$, $R^5$ und $R^6$ jeweils unabhängig voneinander geradkettiges oder verzweigtes Alkyl mit 1 bis 8 C-Atomen oder unsubstituiertes oder substituiertes Aryl ist, bedeuten, sowie die Verwendung der so hergestellten Verbindung zur Herstellung von D-(+)-Biotin.

EP 0 469 432 A1

Die Erfindung betrifft ein verbessertes Verfahren zur Herstellung von chiralen Cyanhydantoinen der Formel I,

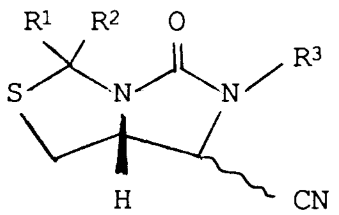

I

wobei

$R^1$ und $R^2$ jeweils unabhängig voneinander H, unsubstituiertes oder substituiertes Alkyl, Cycloalkyl, Aryl, Aralkyl oder Heteroaryl oder zusammengenommen unsubstituiertes Alkylen oder Heteroalkylen, und

$R^3$ H, unsubstituiertes oder durch eine oder mehrere Alkyl-, Alkoxy- oder Alk-2-enyl-Gruppen substituiertes Benzyl oder $SiR^4R^5R^6$,

worin

$R^4$, $R^5$ und $R^6$ jeweils unabhängig voneinander geradkettiges oder verzweigtes Alkyl mit 1 bis 8 C-Atomen oder unsubstituiertes oder substituiertes Aryl sind,

bedeuten.

Der Erfindung lag die Aufgabe zugrunde, ein verbessertes Verfahren für die Herstellung des bicyclischen Cyano-Hyantoins der Formel I, einem wertvollen Zwischenprodukt für die Synthese von optisch aktivem D-(+)-Biotin, bereitzustellen, welches die Durchführung einer Racematspaltung und damit das Verwerfen oder Rückführen des unerwünschten Enantiomeren vermeidet.

Verfahren zur stereospezifischen Synthese von D-(+)-Biotin aus Zuckern geeigneter Konfiguration sind bekannt. So wird in Tetrahedron Letters Nr. 32, S. 2765-2766 (1975), als Ausgangsmaterial D-Mannose, in Agric. Biol. Chem. Nr. 42, S. 465 (1978), D-Glucose und in den DE-OS 31 22 562 und DE-OS 33 20 140 D-Arabinose als chirales Ausgangsmaterial verwendet.

Alle diese Verfahren sind jedoch durch eine hohe Zahl von Syntheseschritten mit folglich geringer Gesamtausbeute gekennzeichnet. Die aufgrund ihrer Zuckernatur meist nicht kristallisierbaren Zwischenstufen werden oft nur in unbefriedigender Reinheit erhalten und erfordern, bedingt durch ihre Polyfunktionalität und der damit verbundenen chemischen Labilität die Einhaltung vergleichsweise enger Reaktionsparameter. Eine Reihe von Zuckern ist auch aus natürlichen Quellen nicht zugänglich, was einen hohen Preis zur Folge hat.

Die Verwendung von L-Cystein, wie aus US-4009172, US-4130713, US-4337345 und Journal of the American Chemical Society Nr. 99, S. 7020 (1977) bekannt, vermeidet zwar die Handhabung labiler Zwischenstufen, führt hingegen über insgesamt 18 Reaktionsstufen unter Abtrennung unerwünschter Isomere nur in unbefriedigender Ausbeute zu optisch aktivem D-(+)-Biotin.

Die von Corey et al. (Tetrahedron Letters 29, 57 (1988)) beschriebene Totalsynthese von D-(+)-Biotin aus dem Hydantoin von L-Cystin ergibt über viele Syntheseschritte lediglich 12 % Ausbeute an D-(+)-Biotin und ist somit für die technische Durchführung nicht geeignet.

In einem weiteren Verfahren werden in Journal of the American Chemical Society Nr. 105, S. 5946 (1983) und in der EP-OS 0 094 776 substituierte 3H,5H-Imidazo [1,5-c]-tetrahydrothiazole beschrieben, aus denen nach Racematspaltung optisch aktives Biotin erhalten wird.

Eine sehr elegante Synthese von D-(+)-Biotin aus Cystein bzw. Cystin über das optisch aktive bicyclische Hydantoin-Derivat der Formel I als Zwischenprodukt wurde von E. Poetsch und M. Casutt (Chimia, 41, 148 (1987) und EP-A2-0 242 686/EP-A2-0 243 734) beschrieben.

Der Nachteil dieser Totalsynthese ist, daß man als Reagenz zur Überführung der Verbindungen der Formel II in die chiralen Cyanhydantoine der Formel I entweder Alkalicyanide oder Trialkylsilylnitrile einsetzen muß.

Bei Verwendung der Alkalicyanide treten Probleme auf wegen ihrer schlechten Löslichkeit, die dazu führen, daß man sie in großem Überschuß einsetzen muß und man die nicht umgesetzten Alkalicyanide im Anschluß an die Reaktion vernichten muß, damit diese hochgiftigen Chemikalien nicht über das Abwasser in die Umwelt gelangen können.

Die Trialkylsilylnitrile können nur in Sicherheitsapparaturen eingesetzt werden, da sie leicht-flüchtig sind

und auch schon bei geringster Feuchtigkeit zu Blausäure hydrolysiert werden. Daher ist eine Anwendung dieses Verfahrens im großtechnischen Maßstab mit Trialkylsilylnitrilen kaum durchführbar.

Dagegen sind die entsprechend den erfindungsgemäßen Verfahren eingesetzten Dialkylaluminiumcyanide leicht handhabbar, gut löslich in allen Lösungsmitteln, in denen sich die Verbindungen der Formel I auch lösen und neigen bei Feuchtigkeit nur im untergeordneten Maße zur Hydrolyse.

Bisher ist lediglich bekannt, daß Dialkylaluminiumcyanide zur Herstellung von Cyanhydrinen (Addition an Carbonylverbindungen z. B. DE 14 93 086) oder von 3-Cyano-substituierten Carbonylverbindungen (Michael-Carbonylverbindungen Addition an 2,3-ungesättigte z. B. JP 75 03 0066-B) eingesetzt werden können. Dabei wurden diese Reaktionen sogar durchgeführt mit Carbonylverbindungen, welche Acyloxy- bzw. Sulphonyloxygruppen aufweisen.

Da die Herstellung der Cyanhydantoine aus den Verbindungen der Formel II mit Alkalicyaniden oder Trialkylsilylnitrilen eine aufwendige Aufreinigung der Reaktionsgemische erforderlich macht, bestand weiterhin Bedarf an einem geeigneten Verfahren zur einfachen ökonomischen Herstellung des bicyclischen Cyan-Hydantoins der Formel I.

Es wurde nun überraschen gefunden, daß das bicyclische Cyano-Hydantoin der Formel I, das ein wertvolles Zwischenprodukt für die Synthese von optisch aktivem D-(+)-Biotin darstellt, durch Umsetzung der Verbindung der Formel II mit einem Dialkylaluminiumcyanid hergestellt werden kann.

Gegenstand der Erfindung ist demnach ein

Verfahren zur Herstellung von Cyanhydantoins der Formel I,

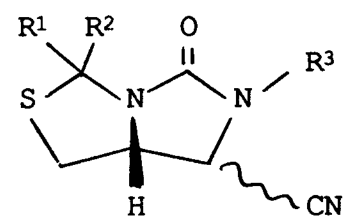

I

wobei

| | |
|---|---|
| $R^1$ und $R^2$ | jeweils unabhängig voneinander H, unsubstituiertes oder substituiertes Alkyl, Cycloalkyl, Aryl, Aralkyl oder Heteroaryl oder zusammengenommen unsubstituiertes Alkylen oder Heteroalkylen, und |
| $R^3$ | H, unsubstituiertes oder durch eine oder mehrere Alkyl-, Alkoxy- oder Alk-2-enyl-Gruppen substituiertes Benzyl oder $SiR^4R^5R^6$, |

wobei

| | |
|---|---|
| $R^4$, $R^5$ und $R^6$ | jeweils unabhängig voneinander geradkettiges oder verzweigtes Alkyl mit 1 bis 8 C-Atomen oder unsubstituiertes oder substituiertes Aryl ist, |

bedeuten,

dadurch gekennzeichnet, daß man eine Verbindung der Formel II,

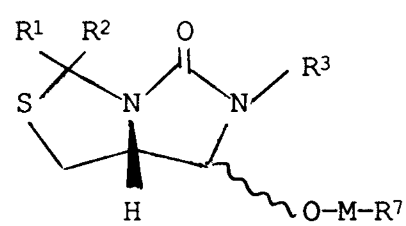

II

wobei

| | |
|---|---|
| $R^1$, $R^2$ und $R^3$ | die angegebene Bedeutung besitzen, und |
| M | CO, CS, SO, $SO_2$ oder S, und |
| $R^7$ | Alkyl oder Perfluoralkyl mit 1 bis 5 C-Atomen, unsubstituiertes oder durch Halogen oder Nitro substituiertes Aryl, Aralkyl oder einen stickstoffhaltigen unsubstituierten, substituierten oder kondensierten Fünfring |

bedeuten,
mit einem Dialkylaluminiumcyanid
wobei

Alkyl geradkettiges oder verzweigtes Alkyl mit 1 bis 6 C-Atomen bedeutet,

umsetzt, insbesondere solch ein Verfahren, wobei

$R^7$ Methyl, Imidazol-1-yl oder Trifluormethyl bedeutet;

weiterhin ein Verfahren wobei man den Ester der Formel II mit Diethylaluminiumcyanid umsetzt.

Darüberhinaus ist die Verwendung des nach dem erfindungsgemäßen Verfahren hergestellten Cyanhydantoins zur Herstellung von D-(+)-Biotin Gegenstand der Erfindung.

Die Herstellung der Ausgangsverbindungen der Formel II erfolgt nach an sich bekannten Methoden, wie sie in der Literatur z,B. Chimia 31, 148 (1987) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Ein bevorzugtes Verfahren zur Herstellung der Verbindungen der Formel II, worin

$M-R^7$ $CO-CH_3$

bedeutet,
besteht darin, einen Alkohol der Formel III,

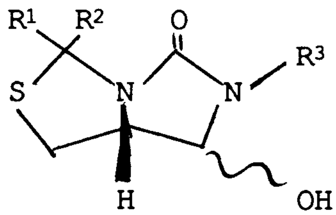

III

worin $R^1$, $R^2$ und $R^3$ die angegebene Bedeutung besitzen, mit Acetanhydrid, gegebenenfalls in einem Verdünnungsmittel, in Gegenwart einer katalytischen Menge eines 4-(N,N-Dialkylamino)-pyridins umzusetzen.

Geeignete Verdünnungsmittel sind insbesondere Kohlenwasserstoffe wie Hexan, Cyclohexan, Benzol oder Toluol und halogenierte Kohlenwasserstoffe wie Dichlormethan, Chloroform oder Tetrachlorkohlenstoff.

Die katalytische Menge an einzusetzendem 4-(N,N-Dialkylamino)-pyridin beträgt etwa 0,5 bis 2,0 Mol-% bezogen auf den Alkohol der Formel III. Dabei sind 0,75 bis 1,5 Mol-% an 4-(N,N-Dimethylamino)-pyridin besonders bevorzugt.

Vor- und nachstehend besitzen $R^1$, $R^2$ und $R^3$ die angegebene Bedeutung, sofern nicht ausdrücklich etwas anderes vermerkt ist.

Bevorzugte Dialkylaluminiumcyanide sind diejenigen, worin die beiden Alkylgruppen mit 1 bis 6 C-Atomen, insbesondere mit 2 bis 4 C-Atomen, gleich sind. Insbesonders bevorzugt sind Dimethylaluminiumcyanid, Diethylaluminiumcyanid, Dipropylaluminiumcyanid, Diisopropylaluminiumcyanid, Diisobutylaluminiumcyanid und Dibutylaluminiumcyanid.

Neben den genannten Dialkylaluminiumcyaniden können auch Alkylaluminiumhalogenocyanide, wie z. B. Ethylaluminiumchlorocyanid, eingesetzt werden.

Die Reste $R^1$ und $R^2$ bedeuten vorzugsweise H, $C_1$-$C_4$-Alkyl, unsubstituiertes oder durch $C_1$-$C_3$-Alkyl und/oder Alkoxy ein- oder mehrfach substituiertes Phenyl oder Benzyl, insbesondere bevorzugt sind gleichzeitig $R^1$ = H und $R^2$ = Phenyl.

$R^1$ und $R^2$ bedeuten weiterhin zusammengenommen unsubstituiertes Alkylen oder Heteroalkylen, vorzugsweise $-(CH_2)_n-$ worin n 3, 4, 5 oder 6 bedeutet oder $-(CH_2)_n-X-(CH_2)_o-$ worin n und o jeweils unabhängig voneinander 1, 2, 3 oder 4, die Summe aus m und o 2, 3, 4, 5 oder 6 und X O, S oder NH bedeuten.

$R^3$ bedeutet vorzugsweise eine Schutzgruppe für das mit ihr verknüpfte Stickstoffatom, die es ermöglicht, die Verbindung der Formel I in ein D-(+)-Biotin-Derivat, dessen Stickstoffatom mit $R^3$ geschützt ist, mit dem erfindungsgemäßen Verfahren überzuführen, und anschließend diese Schutzgruppe unter milden Bedingungen selektiv abzuspalten.

Solche Schutzgruppen sind dem Fachmann geläufig (z.B. Protective Groups in Organic Chemistry, Plenum Press, New York, 1973) und können nach bekannten Methoden eingeführt und abgespalten werden. Bevorzugte Reste $R^3$ sind beispielsweise Benzyl, 1-Phenylethyl, Methoxybenzyl, 3,4-Dimethoxybenzyl, 4-Methylbenzyl, Allyl, Methallyl, Crotyl, Trimethylsilyl oder tert.-Butyldimethylsilyl, Diphenylmethyl, Trityl, 9-H-Fluorenyl, 9-Phenyl-9-Fluorenyl, Methoxymethyl.

Die Durchführung der erfindungsgemäßen Reaktion ist an sich einfach. Die Verbindung der Formel II wird, gegebenenfalls im Gemisch mit einem Verdünnungsmittel, mit dem Dialkylaluminiumcyanid, vorzugsweise in einer Inertgasatmosphäre, versetzt und gerührt. Vorzugsweise wird die Reaktion in einen inerten Lösungsmittel, wie z. B. Dichlormethan, Chloroform, Hexan, Cyclohexan, Toluol, Benzol, Diethylether oder THF durchgeführt. Die Reaktionstemperatur liegt üblicherweise zwischen -50 °C und 150 °C, vorzugsweise zwischen 0° und 50 °C, insbesondere bei Raumtemperatur. Man kann die Reaktion bei Normaldruck, Unterdruck oder Überdruck durchführen, vorzugsweise führt man sie bei Normaldruck oder leichtem Überdruck des Inertgases durch.

Bei diesen Reaktionsbedingungen ist die Reaktion üblicherweise nach 15 Minuten bis 4 Stunden beendet.

Eine bevorzugte Ausführungsform des erfindungsgemäßen Verfahren ist es, die Verbindung der Formel III mit Acetanhydrid in Gegenwart von Dimethylaminopyridin in einem inerten Lösungsmittel umzusetzen und anschließend Dialkylaluminiumcyanid in einem inerten Lösungsmittel zuzusetzen.

Das erfindungsgemäße Verfahren erlaubt somit die Herstellung von optisch aktiven Cyanhydantoinen der Formel I, die nach der in EP-A2-0242686/EP-A2-0243734 beschriebenen Methode zu D-(+)-Biotin weiterverarbeitet werden können, auf einfache und stereospezifische Weise in hohen Ausbeuten aus leicht zugänglichen, wohlfeilen Ausgangsstoffen in wenigen Syntheseschritten und stellt damit einen wesentlichen Fortschritt auf dem Gebiet der Biotinsynthese dar.

Die folgenden Beispiele sollen das erfindungsgemäße Verfahren näher erläutern ohne es zu begrenzen.

Die spezifischen Drehwerte sind auf einem Perkin-Elmer Polarimeter im jeweils angegebenen Lösungsmittel gemessen.

Die Säulenchromatographischen Trennungen werden auf Silica 60 230-400 mesh durchgeführt.

**Beispiel 1**

(7RS,7aR)-3-Phenyl-6-benzyl-7,7a-dihydro-7-acetyloxy-1H,3H-imidazo[1,5-c]thiazol-5(6H)-on

Man suspendiert 32,64 g (0,1 Mol) (7RS, 7aR)-3-Phenyl-6-benzyl-7,7a-dihydro-7-hydroxy-1H,3H-imidazo[1,5-c]-thiazol-5(6H)-on in 150 ml Toluol, versetzt mit 11,22 g (0,11 Mol) Essigsäureanhydrid sowie 0,12 g (1 m Mol)4-(Dimethylamino)-pyridin und rührt das Gemisch 3 Stunden bei 50 °C.

Anschließend kühlt man auf Raumtemperatur ab, wäscht zweimal mit je 100 ml 1 H Salzsäure und engt unter vermindertem Druck ein.

Man erhält 34,7 g (≙ 94 % d.Th.) (7RS, 7aR)-3-Phenyl-6-benzyl-7,7a-dihydro-7-acetoxy-1H,3H-imidazo-[1,5-c]-thiazol-5(6H)-on als Öl.

$$[\ ]_{365}^{20} = -779°, \ c = 1 \ (\text{Methanol})$$

**Beispiel 2**

(7RS,7aR)-3-Phenyl-6-benzyl-7-cyano-7,7a-dihydro-1H,3H-imidazo[1,5-c]thiazol-5(6H)-on

In eine unter Stickstoff gehaltene Lösung von 36,85 g (0,1 Mol) (7RS, 7aR)-3-Phenyl-6-benzyl-7,7a-dihydro-7-acetoxy-1H,3H-imidazo[1,5-c]thiazol-5(6H)-on in 350 ml Toluol tropft man bei -10 °C in 10 Minuten 100 ml einer einmolaren Lösung von Diethylaluminiumcyanid in Toluol und rührt das Reaktionsgemisch weitere 60 Minuten bei Raumtemperatur.

Anschließend wird bei 0 °C vorsichtig mit 300 ml 1 N Salzsäure versetzt und 30 Minuten gerührt. Die organische Phase trennt man ab, wäscht zweimal mit je 200 ml Wasser und engt unter vermindertem Druck ein.

Nach Chromatographie des Rückstandes an Kieselgel (Toluol/Ethylacetat 9 : 1, v/v) erhält man 32,3 g (= 92 % d.Th.) (7RS, 7aR)-3-Phenyl-6-benzyl-7-cyano-7,7a-dihydro-1H,3H-imidazo[1,5-c]thiazol-5(6H)-on als Öl.

$$[\alpha]_{365}^{20} = -807°, \ c = 1 \ (Methanol)$$

**Beispiel 3**

Man suspendiert 32,64 g (0,1 Mol) (7RS, 7aR)-3-Phenyl-6-benzyl-7,7a-dihydro-7-hydroxy-1H,3H-imidazo[1,5-c]thiazol-5(6H)-on in 300 ml Toluol, versetzt mit 11,22 g (0,11 Mol) Essigsäureanhydrid sowie 0,12 g (1 m Mol) 4-(Dimethylamino)-pyridin und rührt das Gemisch 1 Stunde bei 65 °C.

Anschließend tropft man 110 ml einer einmolaren Lösung von Diethylaluminiumcyanid in Toluol ohne Kühlung zu und rührt 1 Stunde nach.

Danach wird bei 20 °C vorsichtig mit 300 ml 1 N Salzsäure versetzt und 60 Minuten kräftig gerührt.

Die organische Phase trennt man ab, wäscht mehrmals mit Wasser und engt unter vermindertem Druck ein. Nach Chromatographie des Rückstandes an Kieselgel (Toluol/Ethylacetat 9 : 1, v/v) erhält man 30,5 g (≙ 87 % d.Th.) (7RS, 7aR)-3-Phenyl-6-benzyl-7-Cyano-7,7a-dihydro-1H,3H-imidazo[1,5-c]thiazol-5(6H)-on als Öl.

$$[\alpha]_{365}^{20} = -813°, \ c = 1 \ (Methanol)$$

**Anwendungsbeispiel 1**

Aus 134,17 g (0,4 mol) (7RS,7aR)-3-Phenyl-6-benzyl-7cyano-7,7a-dihydro-1H,3H-imidazo[1,5-c]thiazol-5(6H)-on erhält man durch Behandeln mit 1000 ml konzentrierter Salzsäure bei 80 °C für 3 Stunden 127,2 g (7RS,7aR)-3-Phenyl-5(6H)-oxo-6-benzyl-7,7a-dihydro-1H,3H-imidazo[1,5-c]thiazol-7-carbonsäure. Diese wird bei 80 °C in 1500 ml wasserfreier Essigsäure mit 80 g Zinkpulver umgesetzt. Nach Zugabe von 41 g Natriumacetat in Dimethylformamid und Erhitzen auf 110 °C erhält man 88 g (3aS,6aR)-1,3-Dibenzyl-tetrahydro-thieno[3,4-d]imidazol-2(3H),4-dion mit einem Schmelzpunkt von 118 °C, welches z.B. nach DE-PS 20 58 234 oder 23 31 244 in D-(+)-Biotin überführt wird.

**Anwendungsbeispiel 2**

Das Grignard-Reagenz hergestellt aus 3,68 g (30 mmol) 1-Chlor-4-methoxybutan und 0,97 g (40 mmol) Magnesium wird mit 6,71 g (20 mmol) (7RS,7aR)-3-Phenyl-6-benzyl-7-cyano-7,7a-dihydro-1H,3H-imidazo-[1,5-c]thiazol-5(6H)-on, (hergestellt nach Beispiel 2) umgesetzt.

Nach saurer Hydrolyse erhält man 6,6 g (7R,7aR)-3-Phenyl-6-benzyl-7-(5-methoxypentanoyl)-7,7a-dihydro-1H,3H-imidazo[1,5-c]thiazol-5(6H)-on.

Dieses wird analog Anwendungsbeispiel 1 mit 7,06 g (108 mmol) Zinkpulver in 200 ml Essigsäure behandelt und anschließend mit Eisessig/Piperidin auf 100 °C erhitzt.

Nach üblicher Aufarbeitung erhält man 8,1 g (3aS,6aR)-1,3-Dibenzyl-4-(4-methoxybutyliden)-tetrahydro-thieno-[4,5-d]imidazol-2(3H)-on, aus welchem nach DE-PS-20 58 243, EP-PS-0 036 030 und EP-PS 0 084 377 D-(+)-Biotin hergestellt wird.

**Patentansprüche**

1. Verfahren zur Herstellung eines chiralen Cyanhydantoins der Formel I,

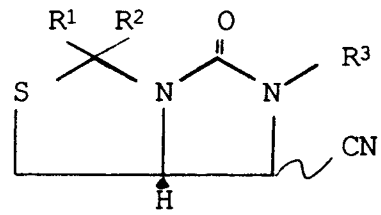

I

wobei

R[1] und R[2] jeweils unabhängig voneinander H, unsubstituiertes oder substituiertes Alkyl, Cycloalkyl, Aryl, Aralkyl oder Heteroaryl oder zusammengenommen unsubstituiertes Alkylen oder Heteroalkylen, und

$R^3$ H, unsubstituiertes oder durch eine oder mehrere Alkyl-, Alkoxy- oder Alk-2-enyl-Gruppen substituiertes Benzyl oder $SiR^4R^5R^6$,

wobei

$R^4$, $R^5$ und R [6] jeweils unabhängig voneinander geradkettiges oder verzweigtes Alkyl mit 1 bis 8 C-Atomen oder unsubstituiertes oder substituiertes Aryl sind,

bedeuten,

dadurch gekennzeichnet, daß man eine Verbindung der Formel II

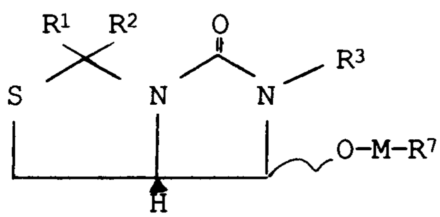

II

wobei

$R^1$, $R^2$ und $R^3$ die angegebene Bedeutung besitzen, und

M CO, CS, SO, $SO_2$ oder S, und

$R^7$ Alkyl oder Perfluoralkyl mit 1 bis 5 C-Atomen, unsubstituiertes oder durch Halogen oder Nitro substituiertes Aryl, Aralkyl oder einen stickstoffhaltigen unsubstituierten, substituierten oder kondensierten Fünfring

bedeuten,

mit einem Dialkylaluminiumcyanid

wobei

Alkyl geradkettiges oder verzweigtes Alkyl mit 1 bis 6 C-Atomen bedeutet,

umsetzt.

**2.** Verfahren nach Anspruch 1, dadurch gekennzeichnet, daß

R7 Methyl, Imidazol-1-yl oder Trifluormethyl bedeutet.

**3.** Verfahren nach Anspruch 1 oder 2, dadurch gekennzeichnet, daß man den Ester der Formel II mit Diethylaluminiumcyanid umsetzt.

**4.** Verfahren nach einem der Ansprüche 1 bis 3, dadurch gekennzeichnet, daß man eine Verbindung der Formel III

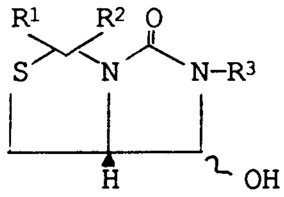

III

worin $R^1$, $R^2$ und $R^3$ die angegebene Bedeutung besitzen, mit Acetanhydrid in Gegenwart von Dimethylaminopyridin in einem inerten Lösungsmittel umsetzt und der Reaktionsmischung ein Dialkylaluminiumcyanid in einer inerten Lösungsmittel zusetzt.

5. Verwendung des nach einem der Ansprüche 1 bis 4 hergestellten chiralen Cyanhydantoins zur Herstellung von D-(+)-Biotin.

Europäisches Patentamt

# EUROPÄISCHER RECHERCHENBERICHT

Nummer der Anmeldung

EP 91112291.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.⁴) |
|---|---|---|---|
| D,A | EP - A2 - 0 242 686 (MERCK PATENT GESELLSCHAFT MIT BESCHRÄNKTER HAFTUNG) * Ansprüche 3-6 * | 1-5 | C 07 D 513/04 |
| D,A | CHIMIA, Band 41, 1987, Basel, E. POETSCH et al. "Stereoselektive Synthesewege zu (+)-Biotin aus L-Cystein." Seiten 148-150 * Seite 149, Zeilen 1-9 * | 1-5 | |
| D,A | DE - A - 1 493 086 (SHINOGI & CO. LTD.) * Anspruch 1 * | 1-4 | |

RECHERCHIERTE SACHGEBIETE (Int. Cl.⁴)

C 07 D 513/00

Der vorliegende Recherchenbericht wurde für alle Patentansprüche erstellt.

| Recherchenort | Abschlußdatum der Recherche | Prüfer |
|---|---|---|
| WIEN | 07-11-1991 | SCHNASS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument